# EUROPÄISCHE PATENTSCHRIFT

(11) **EP 2 674 129 B1**
(45) Veröffentlichungstag und Bekanntmachung des Hinweises auf die Patenterteilung: **27.03.2019**
(21) Anmeldenummer: 13170659.0
(22) Anmeldetag: 05.06.2013
(51) Int. Cl.: A61C 13/00

(54) **HERSTELLUNG INDIVIDUELLER DENTALER PROTHESEN VIA CAD/CAM UND RAPID MANUFACTURING/RAPID PROTOTYPING AUS DIGITAL ERHOBENEN DATEN DER MUNDSITUATION**
PRODUCTION OF INDIVIDUAL DENTAL PROSTHESES VIA CAD/CAM AND RAPID MANUFACTURING/RAPID PROTOTYPING FROM DIGITALLY RECORDED ORAL DATA
FABRICATION DE PROTHÈSES DENTAIRES INDIVIDUELLES VIA CAD/CAM ET RAPID MANUFACTURING/RAPID PROTOTYPING À PARTIR DE DONNÉES SUR LA SITUATION BUCCALE COLLECTÉES PAR VOIE NUMÉRIQUE

(30) Priorität: 11.06.2012 DE 102012011371
(43) Veröffentlichungstag der Anmeldung: 18.12.2013
(73) Patentinhaber: Kulzer GmbH, 63450 Hanau (DE)
(72) Erfinder: Böhm, Uwe, 63456 Hanau (DE); Ruppert, Klaus, 63477 Maintal (DE); Beyer, Mario, 61350 Bad Homburg (DE)
(74) Vertreter: Bendele, Tanja

(56) Entgegenhaltungen:
- EP-A2- 0 348 061
- WO-A1-00/69357
- WO-A1-2012/041329
- DE-A1-102009 056 752
- US-A- 5 605 459
- US-A1- 2004 243 361
- US-A1- 2006 008 777
- US-A1- 2010 086 899
- US-B1- 7 153 135

## Beschreibung

Die Erfindung betrifft Verfahren zur automatisierten Herstellung von Zahnprothesen, insbesondere die Herstellung individueller dentaler Prothesen via CAD/CAM und Rapid Manufacturing/Rapid Prototyping aus digital erhobenen Daten der Mundsituation.

### Hintergrund

Die Herstellung von Zahnvoll- oder-teilprothesen erfolgt nach an sich bekannten Verfahren. Zu nennen sind z. B. die herkömmlichen Verfahren der Pulver/Flüssigkeitstechnologie, wie sie seit langem bekannt und in der Literatur beschrieben sind (z.B. EP 1 243 230 A2, US 6,881,360 B2 und "Dental Materials" in: Ullmann's Encyclopedia of Industrial Chemistry Copyright 2002 by Wiley-VCH Verlag).

Allgemein sind drei unterschiedliche Hauptmaterialklassen zur Herstellung von totalprothetischen Arbeiten bekannt. Dies sind auf Polymethylmethacrylat (PMMA) basierende Zweikomponentenmaterialien [Handelsprodukte Palapress, Paladur (Heraeus Kulzer, DE), SR 3/60® Quick (Ivoclar, LI), Degupress ® (Degussa-Hüls, DE)]; heißhärtende Materialien [Handelsprodukte sind z.B. Paladon® 65 (Heraeus Kulzer, DE), SR 3/60®, SR Ivocap® (Ivoclar, LI), Lucitone® (Dentsply, US)] sowie thermoplastisch verarbeitbare Spritzgießmassen.

Die thermoplastischen Materialien werden erhitzt und meist über ein Spritzgussverfahren in einen Hohlraum injiziert. Ein bekanntes Verfahren ist "Polyapress"®, welches unter anderem von der Fa. Bredent, Senden (DE), vertrieben wird. Es hat nicht an Versuchen gefehlt, Polymere wie etwa PVC, Polyurethan, Polyamid oder Polycarbonat einzusetzen (Ullmann's aaO 5.1.5. Other Denture Resins.)

Weiter existieren Verfahren, die auf licht- oder mikrowellenhärtenden 1-Komponenten-Materialien aufbauen (z.B. Eclipse von DeguDent; (Ullmann's aaO 5.1.3. Light-Cured Polymers, 5.1.4. Microwave-Cured Polymers).

Aus der Zahntechnik sind außerdem manuelle schichtaufbauende Verfahren bekannt. Diese werden meist in Verbindung mit lichthärtenden Materialien angewandt. Z.B. zur Verblendung von Metallkronen oder zur Erstellung einer Prothese. Vorteile bei diesen Verfahren sind die während des Verfahrens mögliche Kontrolle und die Möglichkeit der Variation der Farben, um möglichst ästhetische zahntechnische Arbeiten zu erhalten.

In DE 10 2009 056 752 A1 wird die separate Herstellung von Zahnbogen und Prothesenbasis/Zahnfleischimitation beschrieben. Die Teile sind zum anschließenden Zusammenkleben vorgesehen: Insbesondere wird dort nach Bereitstellung von Daten aus der digitalen Abdrucknahme oder der Digitalisierung einer herkömmlichen Silikon-Funktionsabformung ein farblich geschichteter Kunststoff- oder Keramik-Zahnbogen erzeugt. Parallel ist das Erstellen und Fertigen einer Zahnfleischimitation vorgesehen. Zahnbogen und Zahnfleisch werden dann mittels etablierter Klebeverfahren fest miteinander verbunden.

Es wurden auch schnelle Prototypherstellung (Rapid-Prototyping) -Verfahren zum Einsatz in der Zahntechnik vorgeschlagen. Dabei wird mit polymerisierbaren Schichten (DE 101 14 290 A1, DE 101 50 256 A1) oder Ink-Jet-Pulverdruck (US 6,322,728 B1) gearbeitet. Rapid Prototyping (deutsch schneller Prototypenbau) ist ein Verfahren zur schnellen Herstellung von Musterbauteilen ausgehend von Konstruktionsdaten. Rapid-Prototyping-Verfahren sind somit Fertigungsverfahren, die das Ziel haben, vorhandene CAD-Daten möglichst ohne manuelle Umwege oder Formen direkt und schnell in Werkstücke umzusetzen. Die für diese Verfahrensgruppe relevante Datenschnittstelle ist das STL-Format. Die unter dem Begriff des Rapid Prototyping seit den 1980er Jahren bekannt gewordenen Verfahren sind in der Regel Urformverfahren, die das Werkstück schichtweise aus formlosem oder formneutralem Material unter Nutzung physikalischer und/oder chemischer Effekte aufbauen.

Weiterentwicklungen auf dem Gebiet der Frästechnik (CAD/CAM-Fräsen) und der generativen Fertigungstechniken schneller Prototypenbau (Rapid Prototyping) und schnelle Fertigung (Rapid Manufacturing) halten Einzug in die Dentalprothetik. Der Begriff Schnelle Fertigung bzw. englisch Rapid Manufacturing bezeichnet Methoden und Produktionsverfahren zur schnellen und flexiblen Herstellung von Bauteilen und Serien mittels werkzeugloser Fertigung direkt aus den CAD-Daten. Verwendete Materialien sind Glas, Metall, Keramik, Kunststoffe und neue Materialien (wie UV härtendes Sol-Gel, siehe z. B. Multi Jet Modeling). Da beim Rapid Manufacturing immer die direkte Herstellung des Endprodukts im Mittelpunkt steht, unterscheidet es sich grundlegend von Rapid Prototyping und Rapid Tooling (Schneller Werkzeugbau). Allerdings wird der Begriff "Rapid Prototyping" für gewöhnlich auch gebraucht, wenn eigentlich "Rapid Manufacturing" gemeint ist.

Grundlage dafür ist die digitale Erfassung der Mundsituation durch digitalisierte Abdrucknahme, Direkte (z.B. 3D Kameras) und indirekte Methoden (z.B. Scannen von Modellen) dafür sind an sich bekannt. Kommerziell im Einsatz sind Scan-Technologien wie Lava® C.O.S. von 3M Espe, Bluecam® von Sirona, Hint ELS® directScan oder cara® TRIOS von Heraeus Kulzer. Die Verarbeitung der erhaltenen Daten in virtuellen Artikulatoren ermöglicht die Aufstellung virtuell als Datensatz vorhandener Zähne. Resultat sind Datensätze für individuelle Total- oder Teil-Zahnprothesen. Entsprechende Verfahren werden z.B. in EP 1 444 965 A2 beschrieben, ebenso wie die sich anschließenden Herstellung von Zahnprothesen:
"Nach Abschluss der Arbeit am virtuellen Modell kann direkt die Übertragung auf die Prothese erfolgen, d.h., nach den Daten der virtuellen Zahnaufstellung wird eine Prothesenbasis mit Positionierhilfen für die Zähne hergestellt, in die dann lediglich die betreffenden ausgewählten vorkonfektionierten Zähne eingesetzt zu werden brauchen.
Die Prothesenbasis kann direkt erzeugt werden, oder man kann eine Gussform für sie herstellen. Als Verfahren bieten sich etwa Fräsen oder Rapid-Prototyping an."

### Beispiele für Rapid Manufacturing Techniken sind:

Stereolithographie (SLA), Fused Deposition Modeling (FDM), Selective Laser Sintering (Schichtaufbau durch Sintern von Pulver), Selective Laser Melting (SLM, Schichtaufbau durch vollständiges Umschmelzen und Erstarrenlassen von Pulver), 3D/Inkjet Printing.

In US 7153135 B1 sind diese Verfahren im Einzelnen beschrieben, und zusätzlich Techniken wie "Laminated Object Manufacturing" (u.a. das Schichten keramischer Grünfolien) und "Solid Ground Curing" (Lichthärtung ganzer Schichten durch Schablonen hindurch, besonders geeignet für große Gegenstände). "Ink-Jet Printing" ist dort als Oberbegriff definiert, der das klassische am MIT entwickelte 3D Printing (3DP) und weiterentwickelte Verfahren mit 2 Strahlen (einer bringt Thermoplasten aus, der andere stützendes Wachs) umfasst. Neuere Generationen von Jet-Systemen haben viele Druckköpfe, z.B. 96 (Fa. 3D Systems). Damit können bei einem Überlauf ganze Schichten eines Produkts aufgebracht werden. Ist der Querschnitt des Produkts zu groß, macht die Maschine mehrere Überläufe nebeneinander.

Die vorstehenden Verfahren werden hinsichtlich der Technik und der eingesetzten Materialien ständig verfeinert, so dass sich die anfangs nicht zufriedenstellenden ästhetischen Eigenschaften verbessern. Insbesondere kann man inzwischen nicht nur einzelne und damit einfarbige Ausgangsmaterialien einsetzen. Dadurch werden z.B. beim Herstellen künstlicher Zähne mehrfarbige Einzelbausteine oder ein ineinander Übergehen der Schichtung möglich, und die natürliche Anmutung des Endprodukts lässt sich imitieren.

Die WO2011/077175 A1 offenbart die Herstellung mehrfarbiger Prothesen mittels FDM. Im Bereich der CAD/CAM-Fräs-Technik, im Folgenden vereinfacht als CAD/CAM bezeichnet, ist es bereits heute möglich, mehrfarbige, geschichtete Kunststoff- (z.B. Vita CAD-temp multicolor) oder auch Keramikmaterialien (z.B. Vitablocs Triluxe) zu verarbeiten, die den fertigen Zahn, bzw. die fertige prothetische Arbeit sehr natürlich erscheinen lassen.

### Aufgabenstellung

Es stellt sich die Aufgabe, Verfahren anzugeben, mit denen die automatisierte Herstellung noch weiter verbessert werden kann. Auch soll die Herstellung ästhetisch anspruchsvoller Zahnprothesen mit farblicher Schichtung oder Farb- oder Transparenzabstufungen ermöglicht werden.

### Darstellung der Erfindung

Die Aufgabe wird durch die Merkmale der Ansprüche 1 und 2 gelöst. Bevorzugte Ausgestaltungen sind den weiteren Ansprüchen zu entnehmen.

Gegenstand der Erfindung ist ein Verfahren zur Herstellung einer Totalprothese durch
A) Bereitstellen von 3D-Daten der Mundsituation im zahnlosen Zustand,
B) Digitale Konstruktion der Prothesenbasis für jeweils Unter- und Oberkiefer,
C) Digitale Aufstellung von virtuellen Zähnen mit passender Okklusion und nach ästhetischen Kriterien ausgewählter Zahnform,
   D1) Herstellung des Zahnbogens mit einem automatisierten Verfahren aus den Gruppen der schichtaufbauenden und der materialabtragenden Verfahren,
   D2) Herstellung der Prothesenbasis mit einem automatisierten Verfahren aus den Gruppen der schichtaufbauenden und der materialabtragenden Verfahren,
wobei in Schritt D1 das Innere der Zähne oder des Zahnbogens mit einem automatisierten Verfahren hergestellt wird, ausgewählt aus Stereolithographie (SLA) mit einem Material 2A, Inkjet Printing mit einem Material 2B oder CAD/CAM Fräsen mit einem Material 1B und darauf als Außenschicht mindestens ein zweites Material mit einem automatisierten Verfahren aufgebracht wird, mitttels Fused Deposition Modelling (FDM).

Ferner ist Gegenstand der Erfindung ein Verfahren zur Herstellung einer Teilprothese durch
A) Bereitstellen von 3D-Daten der Mundsituation im teilbezahnten Zustand
B) Digitale Konstruktion der Prothesenbasis
C) Digitale Aufstellung von virtuellen Zähnen mit passender Okklusion
   D1) Herstellung des Zahnbogens mit einem automatisierten Verfahren aus den Gruppen der schichtaufbauenden und der materialabtragenden Verfahren,
   D2) Herstellung von Stützstrukturen oder Befestigungselemente mit einem automatisierten Verfahren aus den Gruppen der schichtaufbauenden und der materialabtragenden Verfahren wobei in Schritt D1 das Innere der Zähne oder des Zahnbogens mit einem automatisierten Verfahren hergestellt wird, ausgewählt aus Stereolithographie (SLA) mit einem Material 2A, Inkjet Printing mit einem Material 2B oder CAD/CAM Fräsen mit einem Material 1B und darauf als Außenschicht mindestens ein zweites Material mit einem automatisierten Verfahren aufgebracht wird, mittels Fused Deposition Modelling (FDM).

In bevorzugten Ausführungsformen des Verfahren erfolgt D2) die Herstellung der Prothesenbasis mit einer der Methoden SLA, Inkjet Printing, FDM, SLM und CAD/CAM-Fräsen aus jeweils für die Methode geeignetem Material 1 oder Material 2.

In einer weiteren bevorzugten Ausführungsform des Verfahren erfolgt D2) die Herstellung der Stützstrukturen oder Befestigungselemente mit einer der Methoden: SLM oder CAD/CAM Fräsen aus jeweils für die Methode geeignetem Material 1. Z.B. Metall oder Hochleistungskunststoff.

### Im Einzelnen sind geeignete Materialien:

Material 1A für SLM: Ein Mitglied der Gruppe: Pulverförmige Stoffe (thermoplastische Kunststoffe) oder Metallpulver, besonders Co Cr Ni-Basislegierungen, edelmetallhaltige Legierungen, besonders die auf dem Dentalgebiet üblichen, nichtrostender Stahl, Titan, thermoplastische Hochleistungspolymere wie PEEK, gefüllte Thermoplaste;
Material 1B Für CAD/CAM Fräsen: Ein Mitglied der Gruppe: Edelmetalle und deren Legierungen, Keramik, besonders Zirkondioxid-Keramik, Polymere, Titan, niedrigschmelzende Legierungen, thermoplastische Hochleistungspolymere wie z.B. PEEK gefüllte Thermoplaste EM-Legierungen;
Material 2A für SLA: Ein Mitglied der Gruppe: Lichtempfindliche Monomermischungen, anorganisch gefüllt oder ungefüllt;
Material 2B für Inkjet Printing (3-D-Printing): Ein Mitglied der Gruppe: Epoxid-/Acrlyatmonomere oder lichthärtende Monomermischungen, lichtempfindliche Monomermischungen, anorganisch gefüllt oder ungefüllt.
Material 2C für FDM: Ein Mitglied der Gruppe: Thermoplastische Hochleistungspolymere wie Polyetheretherketon (PEEK), gefüllte Thermoplaste.

Je nach den eingesetzten Materialgruppen (Material-1, Material-2) ist die Herstellung verschiedener Produkte begünstigt.Die Stützstrukturen oder Befestigungselemente von Teilprothesen werden bevorzugt aus Metall oder Hochleistungspolymeren gefertigt. Es ist auch möglich, Teilprothesen vollautomatisiert herzustellen, indem die Stützstrukturen mit zahnfarbenem Material beschichtet werden.

Das Verfahren eignet sich auch für implantatgestützte Teil- oder Totalprothesen.

Eine weitere Anwendung ist der Austausch schadhafter Prothesen. Nach hinterlegten Daten der beschädigten Prothese kann einfach eine individuelle neue hergestellt werden.
Selbstverständlich kann das in einem Zentrum erfolgen oder direkt in der Zahntechnikerwerkstatt oder der Zahnarztpraxis - je nachdem, wo die maschinelle Ausstattung vorliegt.

Das Verfahren ist natürlich auch für die Herstellung herausnehmbarer Totalprothesen geeignet.

Besonders vorteilhafte Verfahren sind die folgenden:

### Teilprothese, optional implantatgetragen:

Die Tragekonstruktion wird bevorzugt mit SLM gedruckt, das Zahnfleisch dann ebenfalls mit Selective Laser Melting aus geeigneten thermoplastischen Materialien schichtweise aufgebaut.

### Zahnbogen:

Für die Herstellung von Zahnbögen für Teil- oder Totalprothesen eignen sich besonders Inkjet-Verfahren. Der Aufbau aus mehreren Schichten ermöglicht eine Farb-oder Transparenzabstufung.

### Prothesenbasis:

Diese lässt sich vorteilhaft mit Selective Laser Melting aufbauen, vorzugsweise aus Polyacrylaten oder Polymethylmethacrylat.

### Totalprothese:

Zahnbogen und Zahnfleisch werden vorteilhaft separat hergestellt.
Für den Zahnbogen eignen sich die Verfahren
SLA, Inkjet, SLM und FDM und CAD/CAM Fräsen;
für das Zahnfleisch SLA, Inkjet und SLM.

Es ist auch möglich, Einzelzähne oder Zahnbögen durch separaten Aufbau eines Innenteils herzustellen, das nachfolgend außenherum mit mindestens einem weiteren Material verkleidet wird. Die äußere Schicht kann dabei einen von der inneren verschiedenen Transparenzwert haben. Das ermöglicht eine natürliche Anmutung und eignet sich besonders für Frontzähne. Die Außenschicht kann auch mechanisch oder gegen Abrasion besonders stabil sein. Dies wiederum eignet sich besonders für Molare mit starker Kaubelastung.

Technisch kann das dadurch verwirklicht werden, dass das Innere des Zahns mit SLA oder Inkjet aufgebaut wird. Das äußere zweite Material kann z.B. mit FDM aufgebracht werden. Auf diese Weise können auch Antiplaque-Schichten vorgesehen sein.

Die Vorteile der genannten automatisierten Verfahren sind der Zeitvorteil, die größere Genauigkeit - die Passgenauigkeit des fertigen Zahnersatzes - und Reproduzierbarkeit, etwa beim Ersatz beschädigter Zahnprothesen.

Handelt es sich um SLM Verfahren, so ist besonders darauf hinzuweisen, dass dabei die Materialien restmonomerfrei sind, denn es findet nur eine Umformung durch Schmelzen statt. Auch im Fall von Acrylaten werden prinzipiell nur MMA freie höhermolekulare Acrylate eingesetzt. Diese Materialien haben auch Vorteile, was die Arbeitssicherheit in Industriehallen betrifft.

Die Schritte D1) und D2) können auf zwei verschiedenen Maschinen erfolgen, jeweils für rot (Zahnfleisch) und weiß (Zähne).

Eine Wachseinprobe entfällt naturgemäß. Dadurch wird das Verfahren kostengünstiger. Insgesamt ist die Herstellung (von Scan bis zur Einprobe) im Vergleich zu handwerklicher Herstellung schneller.

### Abbildungen

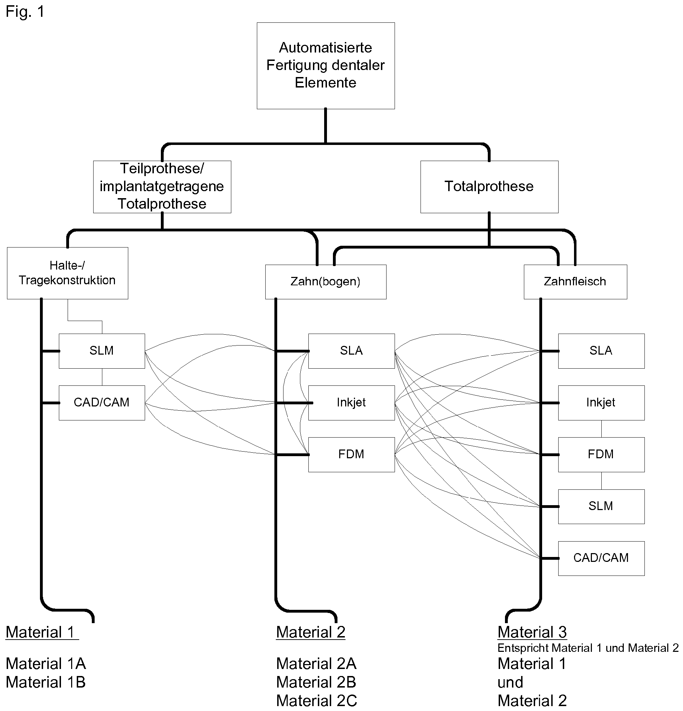

Das Fließbild der Fig. 1 verdeutlicht die Möglichkeiten für verschiedene Ausführungsformen des erfindungsgemäßen Herstellverfahrens und die im Text erläuterten Materialgruppen.

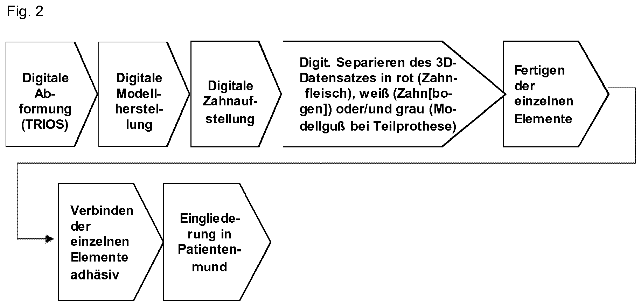

Die in Fig. 2 dargestellten Schritte einer Ausführungsform des erfindungsgemäßen Verfahrens sind im Einzelnen:
- Abformung durch den Zahnarzt über Intraoralscanner
- Erzeugung digitaler Modelldaten
- Optional: Weitergabe des digitalen Modells an das Labor
   digitale Zahnaufstellung
- Optional: Digitales Separieren des 3D Datensatzes in rot (Zahnfleisch), weiß (Zahn[bogen]) oder/und grau (mit grau wird das Innere einer verblendeten Brückenkonstruktion oder der frühere Modellguss bei einer gegebenenfalls implantatgetragenen Teilprothese bezeichnet)
- Fertigen der einzelnen Elemente, ggf. mit Verbindungselementen
- wobei das Innere der Zähne oder des Zahnbogens mit einem automatisierten Verfahren hergestellt wird und darauf als Außenschicht mindestens ein zweites Material mit einem automatisierten Verfahren aufgebracht wird (vorzugsweise wird das Innere der Zähne (Kern) mittels Fräsen oder SLA oder Inkjet-Printing hergestellt und darauf mindestens ein zweites Material mit FDM aufgebracht),
- Verbinden der einzelnen Elemente, bevorzugt adhäsiv
- optional: Nachbearbeitung wie z.B. Einschleifen und Polieren
- Auslieferung an den Kunden.

## Patentansprüche

1. Verfahren zur Herstellung einer Totalprothese durch
A) Bereitstellen von 3D-Daten der Mundsituation im zahnlosen Zustand.
B) Digitale Konstruktion der Prothesenbasis für jeweils Unter- und Oberkiefer,
C) Digitale Aufstellung von virtuellen Zähnen mit passender Okklusion und nach ästhetischen Kriterien ausgewählter Zahnform,
D1) Herstellung des Zahnbogens mit einem automatisierten Verfahren aus den Gruppen der schichtaufbauenden und der materialabtragenden Verfahren,
D2) Herstellung der Prothesenbasis mit einem automatisierten Verfahren aus den Gruppen der schichtaufbauenden und der materialabtragenden Verfahren, wobei in Schritt D1 das Innere der Zähne oder des Zahnbogens mit einem automatisierten Verfahren hergestellt wird, ausgewählt aus Stereolithographie (SLA) mit einem Material 2A, Inkjet Printing mit einem Material 2B oder CAD/CAM Fräsen mit einem Material 1B und darauf als Außenschicht mindestens ein zweites Material mit einem automatisierten Verfahren aufgebracht wird, mittels Fused Deposition Modelling (FDM).

2. Verfahren zur Herstellung einer Tellprothese durch
A) Bereitstellen von 3D-Daten der Mundsituation im teilbezahnten Zustand
B) Digitale Konstruktion der Prothesenbasis
C) Digitale Aufstellung von virtuellen Zähnen mit passender Okklusion
D1) Herstellung des Zahnbogens mit einem automatisierten Verfahren aus den Gruppen der schichtaufbauenden und der materialabtragenden Verfahren,
D2) Herstellung von Stützstrukturen oder Befestigungselementen mit einem automatisierten Verfahren aus den Gruppen der schichtaufbauenden und der materialabtragenden Verfahren wobei in Schritt D1 das Innere der Zähne oder des Zahnbogens mit einem automatisierten Verfahren hergestellt wird, ausgewählt aus Stereolithographie (SLA) mit einem Material 2A, Inkjet Printing mit einem Material 2B oder CAD/CAM Fräsen mit einem Material 1B und darauf als Außenschicht mindestens ein zweites Material mit einem automatisierten Verfahren aufgebracht wird, mittels Fused Deposition Modelling (FDM).

3. Verfahren nach Anspruch 1, mit
D2) Herstellung der Prothesenbasis mit einer der Methoden SLA, Inkjet Printing, FDM, SLM und CADICAM-Fräsen aus Jeweils für die Methode geeignetem Material 1 oder Material 2.

4. Verfahren nach Anspruch 2, mit
D2) Herstellung der Stützstrukturen oder Befestigungselemente mit einer der Methoden: SLM oder CAD/CAM Fräsen aus jeweils für die Methode geeignetem Material 1.

5. Verfahren nach einem der vorstehenden Ansprüche, wobei das Innere hergestellt wird mittels Fräsen aus einem Material 1B, ausgewählt aus Edelmetalle und deren Legierungen, Keramik, Polymere, Titan, niedrigschmelzende Legierungen, thermoplastische Hochleistungspolymere oder
mittels SLA aus einem Material 2A, ausgewählt aus lichtempfindliche Monomermischungen, anorganisch gefüllt oder ungefüllt oder
mittels Inkjet Printing aus einem Material 2B, ausgewählt aus Epoxid-/Acrylatmonomere oder lichthärtende Monomermischungen, lichtempfindliche Monomermischungen, anorganisch gefüllt oder ungefüllt.

6. Verfahren nach Anspruch 5, wobei die Keramik Zirkondioxid-Keramik ist.

7. Verfahren nach einem der vorstehenden Ansprüche, wobei das mindestens eine zweite Material, welches mit FDM als Außenschicht auf das Innere des Zahns oder des Zahnbogens aufgebracht wird, ausgewählt wird aus thermoplastischen Hochleistungspolymeren.

8. Verfahren nach einem der vorstehenden Ansprüche, wobei das zweite Material ausgewählt wird aus Polyetheretherketone (PEEK) oder gefüllte Thermoplasten.

9. Verfahren nach mindestens einem der vorstehenden Ansprüche, wobei die Fügung von Zahnbogen und Prothesenbasis über mechanische Elemente wie z.B. Führungsschienen, Nuten und geeignete Retentionselemente oder Kleben oder formschlüssiger Verbund erfolgt.

10. Verfahren nach Anspruch 1 oder 3, wobei die Schritte D1 und D2 mit Inkjet Prlntlng Verfahren erfolgen.

11. Verfahren nach Anspruch 2 oder 4, wobei Schritt D1 mit Inkjet Printing und Schritt D2 mit CAD/CAM Fräsen erfolgt.

## Claims

1. Method for the production of a complete prosthesis by
A) providing 3D data of the oral situation in edentulous state,
B) digital construction of the denture base for lower and upper jaw respectively,
C) digital positioning of virtual teeth with an appropriate occlusion and a tooth shape selected according to aesthetic criteria,
D1) production of the dental arch using an automated method from the groups of layer-building and of material-removing methods,
D2) production of the denture base using an automated method from the groups of layer-building and of material-removing methods,
wherein, in step D1, the inside of the teeth or of the dental arch is produced using an automated method, selected from Stereolithography (SLA) with a material 2A, inkjet printing with a material 2B or CAD/CAM milling with a material 1B, and at least one second material is applied thereon as an external layer using an automated method, by means of Fused Deposition Modelling (FDM).

2. Method for the production of a partial prosthesis by
A) providing 3D data of the oral situation in partially toothed state
B) digital construction of the denture base
C) digital positioning of virtual teeth with appropriate occlusion
D1) production of the dental arch using an automated method from the groups of layer-building and of material-removing methods,
D2) production of support structures or fastening elements using an automated method from the groups of layer-building and of material-removing methods wherein, in step D1, the inside of the teeth or of the dental arch is produced using an automated method, selected from Stereolithography (SLA) with a material 2A, inkjet printing with a material 2B or CAD/CAM milling with a material 1B, and at least one second material is applied thereon as an external layer using an automated method, by means of Fused Deposition Modelling (FDM).

3. Method according to claim 1, with
D2) production of the denture base using any one of the methods SLA, inkjet printing, FDM, SLM, and CAD/CAM milling from a material 1 or material 2 being suitable for the method respectively.

4. Method according to claim 2, with
D2) production of the support structures or fastening elements using any one of the methods: SLM or CAD/CAM milling from a material 1 being suitable for the method respectively.

5. Method according to any one of the preceding claims, wherein the inside is produced by means of milling from a material 1B, selected from precious metals and alloys thereof, ceramic, polymers, titanium, low-melting alloys, thermoplastic high-performance polymers, or
by means of SLA from a material 2A, selected from light-sensitive monomer mixtures, filled with inorganic substances or non-filled, or
by means of inkjet printing from a material 2B, selected from epoxy/acrylate monomers or light-curing monomer mixtures, light-sensitive monomer mixtures, filled with inorganic substances or non-filled.

6. Method according to claim 5, wherein the ceramic is a zirconium dioxide ceramic.

7. Method according to any one of the preceding claims, wherein the at least one second material which is applied onto the inside of the tooth or of the dental arch as an external layer using FDM, is selected from thermoplastic high-performance polymers.

8. Method according to any one of the preceding claims, wherein the second material is selected from polyetheretherketones (PEEK) or filled thermoplastics.

9. Method according to at least one of the preceding claims, wherein joining of dental arch and denture base is implemented via mechanical elements, such as, e.g., guide rails, grooves, and suitable retention elements, or via gluing or form-fitting connection.

10. Method according to claim 1 or 3, wherein steps D1 and D2 are implemented by inkjet printing methods.

11. Method according to claim 2 or 4, wherein step D1 is implemented by inkjet printing and step D2 is implemented by CAD/CAM milling.

## Revendications

1. Procédé pour la production d'une prothèse totale par
A) fournissant des données 3D de la situation orale en état édenté,
B) construction numérique de la base de la prothèse pour la mâchoire inférieure et supérieure respectivement,
C) positionnement numérique des dents virtuelles avec une occlusion appropriée et une forme de dent sélectionnée selon des critères esthétiques,
D1) production de l'arcarde dentaire utilisant une méthode automatisée des groupes des méthodes de construction de couche et d'enlèvement de matière,
D2) production de la base de la prothèse utilisant une méthode automatisée des groups des méthodes de construction de couche et d'enlèvement de matière,
selon lequel, en étape D1, l'intérieure des dents ou de l'arcade dentaire est produit utilisant une méthode automatisée, sélectionnée de la stéréolithographie (SLA) avec une matière 2A, l'impression jet d'encre avec une matière 2B ou le fraisage CAO/FAO avec une matière 1B, et au moins une deuxième matière est appliquée là-dessus en tant qu'une couche extérieure utilisant une méthode automatisée, au moyen de la modélisation par dépôt fusionné (FDM).

2. Procédé pour la production d'une prothèse partielle par
A) fournissant des données 3D de la situation orale en état denté partiellement
B) construction numérique de la base de la prothèse
C) positionnement numérique des dents virtuelles avec une occlusion appropriée,
D1) production de l'arcarde dentaire utilisant une méthode automatisée des groupes des méthodes de construction de couche et d'enlèvement de matière,
D2) production des structures de support ou des éléments de fixation utilisant une méthode automatisée des groups des méthodes de construction de couche et d'enlèvement de matière,
selon lequel, en étape D1, l'intérieure des dents ou de l'arcade dentaire est produit utilisant une méthode automatisée, sélectionnée de la stéréolithographie (SLA) avec une matière 2A, l'impression jet d'encre avec une matière 2B ou le fraisage CAO/FAO avec une matière 1B, et au moins une deuxième matière est appliquée là-dessus en tant qu'une couche extérieure utilisant une méthode automatisée, au moyen de la modélisation par dépôt fusionné (FDM).

3. Procédé selon la revendication 1, avec
D2) production de la base de la prothèse utilisant l'une des méthodes la SLA, l'impression jet d'encre, la FDM, la SLM, et le fraisage CAO/FAO à parti d'une matière 1 ou d'une matière 2 étant apte à la méthode respectivement.

4. Procédé selon la revendication 2, avec
D2) production des structures de support ou des éléments de fixation utilisant l'une des méthodes : la SLM ou le fraisage CAO/FAO à parti d'une matière 1 apte à la méthode respectivement.

5. Procédé selon l'une des revendications précédentes, selon lequel l'intérieure est produit au moyen du fraisage à partir d'une matière 1B, sélectionnée des métaux précieux et des alliages desquelles, de la céramique, des polymères, du titane, des alliages à bas point de fusion, des polymères thermoplastiques de haute performance, ou
au moyen de la SLA à partir d'une matière 2A, sélectionnée des mélanges de monomères photosensibles, chargés avec des substances inorganique ou non chargés, ou
au moyen de l'impression jet d'encre à partir d'une matière 2B, sélectionnée des monomères d'époxy/acrylate, ou des mélanges de monomères photopolymérisables, des mélanges de monomères photosensibles, chargés avec des substances inorganique ou non chargés.

6. Procédé selon la revendication 5, selon lequel la céramique est une céramique en oxyde de zirconium.

7. Procédé selon l'une des revendications précédentes, selon lequel l'au moins deuxième matière, qui est appliquée sur l'intérieure de la dent ou de l'arcade dentaire en tant qu'une couche extérieure utilisant la FDM, sélectionné des polymères thermoplastiques de haute performance.

8. Procédé selon l'une des revendications précédentes, selon lequel la deuxième matière est sélectionnée du polyétheréthercétone (PEEK) ou de la matière thermoplastique chargée.

9. Procédé selon au moins une des revendications précédentes, selon lequel l'assemblage de l'arcade dentaire et de la base de la prothèse est effectuée via des éléments mécaniques, tels que, p.ex. des rails de guidage, des rainures et des éléments de rétention appropriés, ou via le collage ou la liaison de forme.

10. Procédé selon la revendication 1 ou 3, selon lequel les étapes D1 et D2 sont effectuées par des méthodes d'impression jet d'encre.

11. Procédé selon la revendication 2 ou 4, selon lequel l'étape D1 est effectuée par l'impression jet d'encre et l'étape D2 est effectuée par le fraisage CAO/FAO.
